# EUROPEAN PATENT APPLICATION

(11) **EP 0 972 841 A1**
(43) Date of publication of application: **19.01.2000**
(21) Application number: 99201592.5
(22) Date of filing: 20.05.1999
(51) Int. Cl.: C12N 15/87, C12N 15/86, C12N 7/04, A61K 47/48, C12N 15/10

(54) **Display of viral proteins as ligands for cell-surface receptor**

(30) Priority: 20.05.1998 EP 98201678
(71) Applicant: Introgene B.V., 2333 AL Leiden (NL)
(72) Inventor: Van Es, Helmuth, 2133 DJ Hoofddorp (NL)
(74) Representative: Ottevangers, Sietse Ulbe

(57) **Abstract**

The invention relates to the targeted delivery of substances to cells. The invention provides a method for selecting at least one mutant protein derived from a viral protein as a ligand capable of binding to a cell-surface receptor, comprising displaying at least one mutant of said protein on the surface of a micro-organism expressing said protein, further comprising selecting at least one such a mutant micro-organism for its capacity to bind to said receptor further comprising incorporating said protein in a virus-like particle or gene-delivery vehicle allowing targeting of said virus-like particle or gene-delivery vehicle to cells carrying said receptor.

## Description

The invention relates to the targeted delivery of substances to cells.

Delivery of substances to cells allows specific treatment of said cells with compounds that act in the targeted cell. For example, tumour cells, when targeted with toxic components, selectively die when said toxin is delivered to said cell. Yet other cells, when provided with a gene lacking in said cell, can be restored in their function, so-called gene therapy.

Delivery of a compound to a cell preferably occurs with a vehicle or particle that effectively brings the compound to the desired cell or cells and than delivers said compound into that cell (in vivo or in vitro) where it can exert its action. For this purpose, for example particles such as virus-like particles are suited. These particles, often derived from known viruses, such as retrovirus or adenovirus, are small enough to penetrate in-between tissues and cells and arrive at a cell of choice where it for example can fuse with said cell and deliver its compound. Said virus-like particles may or may not be infectious in themselves, their main concern is the targeted delivery of the compound of interest, such as a gene, a toxin or immunostimulating components such as antigens. Yet other examples are gene-delivery vehicles, specifically designed to transfer a gene to a cell of interest. Virus-like particles capable of delivering a gene are examples of said gene-delivery vehicles, however, also other examples of such vehicles, of non-viral origin, such as liposomes or microbodies, or even latex particles, are known. Vehicles such as liposomes or microbodies can of course also carry other compounds than a gene, in-particular toxic or immunostimulating components such as antigens can be included in such a vehicle.

These vehicles or particles all have in common that they need to be provided with a molecule or fragment thereof (ligand) capable of binding with said targeted cell, allowing targeting of said particle or vehicles to cells. There is a need for specific or broadly applicable ligands that react with cell-surface receptors on cells. In particular there is a need for ligands that react with cell-surface receptors after which efficient transfer of said compound to said cell, such as a gene, is possible. Especially in human medicine, such a ligand would enable better application of gene-transfer therapy than is possible now.

It has been a long-standing objective to exploit retrovirus technology in human gene therapy applications. However, the infection spectrum of retroviruses clearly limits the applications of these viruses in such applications. All known env variants have a rather broad infection spectrum in common. Here lies one of the major shortcomings of current recombinant retrovirus technology. For the purpose of gene therapy, retroviruses are very useful vehicles for the transfer of therapeutic sequences, if proper ligand-receptor targets were available. The concept of the use of retroviruses or adenoviruses in human gene therapy is well documented. However, it would be clearly advantageous and desirable to devise a strategy for targeted delivery of virus-like particles or gene delivery vehicles and modification of the infection spectrum.

The invention provides a method for selecting at least one mutant protein derived from a viral protein as a ligand capable of binding to a cell-surface receptor, comprising displaying at least one mutant of said protein on the surface of a micro-organism expressing said protein, further comprising selecting at least one such a mutant microorganism for its capacity to bind to said receptor. For example provided are materials and methods to develop envelope or capsid molecules that can bind to preferred target cells for gene therapy. In order to obtain a novel protein capable of binding a chosen target, DNA molecules, each encoding for example a envelope molecule or parts thereof comprising one of a family of similar potential binding domains and a structural signal calling for the display of the protein on the outer surface of a micro-organism, such as a bacterial cell, bacterial spore or phage (the genetic package) are introduced into that genetic package. The protein or part thereof is expressed and the potential binding domains displayed on the outer surface of the package. The micro-organisms, cells or viruses bearing the binding domains which recognise the target receptor molecule are isolated and amplified. The successful binding domains of the variant proteins are then characterised. One or more of these successful binding domains are used as a basis for the design of a new family of potential binding domains, and the process is repeated until a novel binding domain having a desired affinity and specificity for the target molecule is obtained.

The invention provides a method according to the invention further comprising incorporating said protein in a virus-like particle or gene-delivery vehicle allowing targeting of said virus-like particle or gene-delivery vehicle to cells carrying said receptor. Mutant proteins find applications in the design of vector systems for entry into cells including human cells. Preferred are those retroviral envelope molecules or virus capsid proteins or parts thereof, which - when incorporated in a virus-like particle or gene delivery vehicle - can infect desired target cells at increased specificity and efficiency.

The invention further provides a method according to the invention wherein said micro-organism is a filamentous phage. Phage display is a powerful technique allowing the efficient screening of large numbers of random sequences in order to select the best binding sequence for any chosen application. The power of phage display is based on the ability of filamentous bacteriophages to easily express and display foreign protein moieties as fusions with phage coat proteins accompanied by the genetic material encoding that foreign protein moiety. Foreign sequences can be inserted at the amino terminus of either gene III (encoding for the minor coat protein) or gene VIII (encoding for the major coat protein) of a filamentous phage. This is usually possible without significantly interfering with the phage's life cycle (Smith, 1985),US patents 5,223,409 and 5,403,484. In one embodiment of the invention the genetic package is a filamentous phage such as a M13 phage, and the protein includes for example the outer surface transport signal of the M13 gene III protein.

The invention further provides a method according to the invention wherein said protein comprises at least a fragment of an envelope protein, preferably derived from a retrovirus. The invention provides retroviral vectors including murine leukemia retroviral and lentiviral vectors. Peptide phage display has been used for the isolation of ligands that can be incorporated in a retroviral envelope to change the tropism of the original envelope and the resulting retroviral vector (Hall et al., 1997). The disadvantage of this approach is that the effect of insertion of a foreign peptide into a retroviral envelope is unpredictable and therefore the general applicability for retroviral vector improvement limited. Therefore functional display of the retroviral envelope itself is highly preferred.

The invention provides functional display of a retroviral envelope mutant on the surface of a filamentous phage. An advantage is that said mutant is presented in its most natural conformation, allowing for unrestrained selection of mutants capable of binding to said receptor. In one embodiment of the invention a potential binding domain or ligand is related to ecotropic Moloney Murine Leukemia envelope protein, the genetic package is M13 phage, and the protein includes the outer surface transport signal of the M13 gene III protein.

In yet another embodiment, the invention provides a method according to the invention wherein said protein comprises at least a fragment of a capsid protein, preferably derived from an adenovirus. Said capsid protein can for example comprise a selected ligand as provided by the invention and be derived from adenovirus icapsid protein including but not limited to the HI loop of the knob domain of an adenovirus, preferably an adenovirus which does not bind to the adenoviral receptor CAR1 or MHC1. This results in an adenovirus that enters cells through a receptor of choice, thereby greatly facilitating the usefulness of a virus-like particle or gene-delivery vehicle derived from adenovirus.

In yet another embodiment of the invention, a method according to the invention is provided wherein said protein comprises a at least a fragment of a coat protein, preferably derived from a bacteriophage.

The invention provides a method according to the invention wherein said cell-surface receptor is an amino acid transporter, preferably a cationic amino acid transporter such as mCAT1. Selection for protein mutants according to the invention with preferred characteristics from phage libraries can be done using *in vitro* or *in vivo* selection methods or a combination of both. Phage libraries displaying mutant envelopes can be subjected to a number of selection rounds on desired target cells or tissues by injecting the libraries intravenously animals such as mice *in vivo* combined with a selection on relevant human tissues. By way of illustration and not intended to be a limitation human chimeric NOD-SCID mice (Dick et al., 1997) which have a functional human hemopoiesis can be used to inject envelope display libraries followed by sampling the bone marrow and isolation of the targeted phages from marrow cells after various periods of time. This is then followed by amplification of the envelope displaying phages in E.coli and repeated *in vivo* selections. The *in vivo* selections can be combined with selections on purified samples of human hemopoietic stem cells including but not limited to CD34^{positive} CD38 ^{negative} or CD34^{positive}(CD33,CD38,CD71)^{negative} cells isolated from human bone marrow, cord blood or growth factor mobilised peripheral blood using flow cytometry (Knaan-Shanzer et al., 1996).

The invention further provides a method wherein said mutant protein is derived mutant retroviral envelopes that are derived from wild-type envelopes including but not limited to ecotropic, amphotropic and GALV retroviral envelopes by employing the disclosed envelope display technology and which employ a novel or better receptor molecule to enter the target cell by binding or improved binding to this novel or native receptor or receptors. These new retroviral envelope molecules, when incorporated in a virus-like particle or gene-delivery vehicle, can bind to receptor positive cells, such as human PHSCs, with increased specificity and efficiency. Using methods described herein other retroviral envelope molecules can be displayed and converted into libraries of mutant envelopes including but not limited to GALV, amphotropic envelope and spleen necrosis viral envelope (Battini et al., 1992),(Dornburg, 1995). The mutant retroviral envelopes can be used to pseudotype recombinant type C retrovirus including but not limited to murine leukemia retroviral vectors. In a preferred example, said mutant protein is derived from glycoprotein 70 (gp70) of Moloney murine leukemia virus; constructs comprising such mutant protein are for example provided in the experimental part of this description.

The invention further provides a virus-like particle or gene-delivery vehicle obtainable by a method according to the invention. Examples of such particles or vehicles are derived from retrovirus, adenovirus or bacteriophage M13, as described above. Said particles or vehicles can further comprise toxins or antigens or other substances that need to be delivered to a cell, be it in vitro or in vivo.

In a preferred embodiment, a virus-like particle or gene-delivery vehicle is provided for use as a gene-transfer vehicle. Another embodiment of this invention is to use the disclosed retroviral envelope display technology to develop particles or vehicles such as retroviral envelope mutants derived from pathogenic retroviruses and which can be used to block productive infection of cells in a human patient. Mutant envelope displaying phages that block a receptor can then be used as such or recombinant soluble mutant protein can be made thereof and used to treat pathogenic virus infections.

Another embodiment of this invention is to employ the envelope mutants made according tot the methods described herein in non-viral gene transfer vehicles including but not limited to incorporation in liposomes and phosphazenes (W097/07226). This can be done by producing mutant envelopes as recombinant proteins or as envelope displaying phages and mixing or chemically linking them with liposomal or phosphazene preparations carrying a DNA expression construct of a therapeutic DNA sequence. Alternatively the retroviral envelope displaying phages itself may be used as the vector for the therapeutic sequence and modified to a DNA vector.

The invention further provides a method for selecting a filamentous phage expressing a protein capable of binding to a ligand comprising constructing a phage library, enriching said library for phages having desired binding characteristics by at least one round of selection of phages for their capacity to bind to a synthetic peptide derived from said ligand, further comprising enriching said library for phages having desired binding characteristics by at least one round of selection of phages for their capacity to bind to a cell expressing said ligand, for example wherein said ligand is a amino acid transporter, preferably mCAT1.

The invention is further explained in the experimental part not limiting the invention thereto.

### Experimental part

Retroviruses are RNA viruses which efficiently integrate their genetic information into the genomic DNA of infected cells via a reverse-transcribed DNA intermediate. This property of their life-cycle and the fact that parts of their genetic material can be replaced by foreign DNA sequences make retroviruses one of the most promising vectors for the delivery of genes in human gene therapy procedures, most notably for gene therapies which rely on gene transfer into tissues where integration of the therapeutic sequence is needed. Most retroviral vector systems are based on mouse retroviruses and consist of two components, i.e. (i) the recombinant retroviral vector carrying the foreign sequences of interest, and (ii) so-called packaging cells expressing the structural viral proteins of which the encoding sequences are lacking in the retroviral vector. Expression of (i) in (ii) results in the production of recombinant retroviral particles capable of transducing susceptible target cells.

The infectivity and host cell range (tropism) of the retrovirus particle is conferred by an envelope glycoprotein expressed in the bilayer of the retroviral particle and which specifically binds to a receptor molecule on the target cell membrane. The envelope glycoprotein of all known retroviruses consists of two associated peptides gp70 and p15 and which are derived by proteolytic cleavage from the same precursor protein encoded by the retroviral envelope (env) gene (Gunzburg and Salmons, 1996; Weiss, 1996). The amino terminal domain gp70 encompasses specific binding site(s) for its receptor on the target cell membrane, determining the virus host range. Within this domain of about 200 amino acids highly conserved sequences are present that are interrupted by two segments designated VRA and VRB which vary in sequence and length among various mammalian type C retroviruses. In addition some retroviral envelopes have an additional variable domain VRC (Battini et al., 1992). The carboxy terminal peptide or pl5 polypeptide is assumed to mediate fusion between the virus membrane and - depending on the type of virus - the plasma membrane or intracellular vesicle membrane of the target cell (Januszeski et al., 1997; Thomas et al., 1997).

Recently also N-terminal sequences have been implied to play a role in membrane fusion in addition to the key determinant of membrane fusion, the pl5 polypeptide (Bae et al., 1997). Besides its role in fusion between the virus membrane and target cell membranes, the carboxy terminal peptide contains trans-membrane anchor sequences for anchoring in the virion bilayer, and is also crucial for the selective uptake of the envelope glycoprotein in the virus particle. The envelope oligomeric complex consisting of gp70 and p15 is expressed on the surface of the virion as a trimer. Trimerization is believed to be crucial for membrane fusion and is mediated by a trimerization domain in p15 on the outside of the virion (Zhao et al., 1997),(Fass et al., 1996). Nevertheless receptor binding has been observed for monomeric soluble forms containing the variable regions of ecotropic and amphotropic gp70 proteins (Davey et al., 1997). It has been a long-standing objective to exploit retrovirus technology in human gene therapy in order to transfer therapeutic sequences. However, the infection spectrum of retroviruses limits the applications of these viruses in such applications. In numerous gene therapy applications improved or targeted delivery of genes into defined cells or tissues would be desired, including *in vitro* gene transfer into cell types that are present in low abundance in cell mixtures and *in vivo* gene transfer into specific cells and tissues of patients and animals.

Several envelope glycoprotein variants with different infection spectra for mammalian cells have been identified (Battini et al., 1992).
Many envelope variants have a rather broad infection spectrum in common or have infection spectra that do not include human or primate cells and thus are not relevant for human gene therapy applications. Those envelope molecules that do infect primate cells when incorporated in a recombinant retroviral vector often transduce their specific target cells with low efficiencies. Here lies one of the major shortcomings of current recombinant retrovirus technology.

Examples of recombinant viruses that infect primate cells are virions that carry amphotropic or GaLV envelopes. Recombinant viruses carrying an amphotropic or GaLV envelope are capable of infecting human and murine cells and are commonly used in gene transfer trials including human gene therapy involving the pluripotent hemopoietic stem cell (PHSC) an important target for human gene therapies (Havenga et al., 1997). Gene transfer frequencies into PHSCs of human patients and non human primate animal models have been shown to be extremely low and limit therapeutic stem cell gene therapy (Havenga et al., 1997; Hoogerbrugge et al., 1996; Van Beusechem et al., 1993; van Beusechem et al., 1992).

One limiting factor has indeed been shown to be low expression levels of retroviral receptors such as the receptor mediating entry of amphotropic MuLV retrovirus (GLVR2) (Orlic et al., 1996; van Es et al., 1996). The quiescent state of PHSCs when isolated for *ex vivo* gene transfer procedures poses another blockade (Knaan-Shanzer et al., 1996). Murine stem cell gene therapy experiments have traditionally been performed with ecotropic MuLV vectors (Havenga et al., 1997). Recombinant viruses carrying an ecotropic envelope are only capable of infecting murine cells. Transfer of genes into murine PHSCs using ecotropic retroviral vectors has been shown to result in high transduction efficiencies in circulating PHSC derived peripheral blood cells (PBL). The transduction efficiencies are high enough to be therapeutic if achieved in human PHSCs, reaching levels of PHSC gene transfer varying between 30-80 %.

A small number of studies have been performed in which the transduction efficiency into murine PHSCs of ecotropic and amphotropic retroviruses were actually compared directly (Havenga et al., 1997). One of these studies indicated that infection with amphotropic retrovirus resulted in expression and thus transgene presence for less than 8 weeks whereas infection with ecotropic virus resulted in expression for more than 44 weeks after transplantation (Demarquoy, 1993). In a similar study, ecotropic virus was shown to be approximately 50 fold more efficient in transducing murine PHSCs as compared to amphotropic retrovirus (Orlic et al., 1996).

Ecotropic and amphotropic retrovirus differ in the receptor that is employed for virus entry (Albritton et al., 1989; van Zeijl et al., 1994). Ecotropic virus binds target cells via the ecotropic receptor mCAT1 which is a transporter of cationic L-amino acids (Kim et al., 1991) and amphotropic retrovirus binds target cells via the amphotropic receptor GLVR2, a sodium dependent phosphate transporter (Kavanaugh et al., 1994; Miller and Miller, 1994; van Zeijl et al., 1994).

Another limitation for the application of retroviral vectors in gene therapy for human diseases is their relative instability during production, down stream processing including purification and storage of final retroviral gene therapeutics or vaccins (Braas et al., 1996). The *in vitro* half life of recombinant murine leukemia retroviral vectors is only in the range of 8 hours (Kaptein et al., 1997). *In vivo* administration of retroviral vectors in particular those based on murine leukemia retrovirus is hampered by inactivation by the immune-system including the complement system and would greatly benefit from technologies enabling rapid improvement of envelopes as envelopes are a prime target for the immune-system (Takeuchi et al., 1994). Retroviral envelopes can be given a defined specificity for a specific ligand by insertion or inclusion of that ligand. Ligands that have been used are synthetic peptides (Hall et al., 1997), growth factors (Cosset et al., 1995), single chain antibodies (Ager et al., 1996; Marin et al., 1996) and immunoglobulin binding ligands such as one of the receptors for immunoglobulins (W097/05266). To produce envelopes that when expressed in the retroviral bilayer membrane give rise to functional, high titer vector preparations is difficult and cumbersome because the effect of insertion of a foreign sequence into the envelope is unpredictable. The envelope of spleen necrosis virus is an envelope that in general allows easy insertion of single chain antibodies but the resulting specificity of the molecule is in doubt (Chu et al., 1994).

A number of mutant ecotropic envelope molecules have been described in the literature. MacKrell et al have mutated amino acids within the receptor-binding domain VRA of ecotropic MuLV envelope in order to identify residues involved in receptor binding. Virions incorporating mutant envelopes carrying mutations at amino acid residue D84 have lost their binding capabilities to the ecotropic receptor mCAT1 (MacKrell et al., 1996). Virions carrying D84 mutated envelope protein were tested on human cells to search for a possible change in receptor recognition specificity but were found not to infect human cells (Mike Januszeski, personal communication).
Skov and Andersen have studied ecotropic Moloney envelope interactions with mCAT1 by generation of mutant envelope molecules with mutated arginine and lysine residues in gp70 including VRA followed by introduction in a replication competent retroviral backbone (Skov and Andersen, 1993). Mutations R135G, K137Q, R157G and R159A (R102G,K104Q,R124G and R126A without signal peptide respectively) resulted in virions that were not able to replicate.
Kingsman et al have described in PCT application WO96/31602 an insertion site in the VRA domain of ecotropic envelope which allows modification of the tropism. An integrin binding sequence was inserted resulting in infection of human cells expressing the respective integrin other peptides inserted at this site did not result in useful molecules.
PVC-211 murine leukemia virus (MuLV) is a neuropathogenic variant of ecotropic Friend MuLV (F-MuLV) that causes a rapidly progressive neurodegenerative disease in susceptible rodents. PVC-211 MuLV, but not the parental F-MuLV, can infect rat brain capillary endothelial cells (BCEC) efficiently, and the major determinant for BCEC tropism of PVC-211 MuLV is localised within the envelope gene. More specific analysis indicated that E116G and E129K substitutions in the background of the F-MuLV envelope envelope protein were sufficient for conferring BCEC tropism on the virus (Masuda et al., 1996a). Host range changes of these mutations were found to include CHO cells normally not infectable with ecotropic F-MuLV or M-MuLV. The latter suggests that these mutations overcome a negative effect of CAT1 CHO cell receptor glycosylation in the region of virus binding in the third extracellular domain of mCAT1 (Masuda et al., 1996b).
In conclusion murine leukemia retroviral envelope mutants revealed sites in the envelope that are important for folding or more importantly for receptor binding but failed to yield variants useful for human gene therapy purposes.

By employing particular natural retroviral envelope variants the transduction spectrum can be limited or expanded to some extend, but true specificity for particular human target cells of interest can not be obtained following the above described strategy (Masuda et al., 1996a; von Kalle et al., 1994; Wilson et al., 1994).

Evolution of retroviruses to variants with preferred tropism characteristics without the use of rational approaches including specific ligand insertions or mutagenesis of specific sites can be done using replication competent retroviruses which are evolved for particular target cells by multiple passages of the virus on those target cells. The disadvantages of this type of approach are: (1) The process is lengthy in time and laborious, (2) dependant on replication and not only on the envelope proteins that need to be evolved or optimized and (3) often the virus needs to have a low but clear affinity for the target cells in question (Taplitz and Coffin, 1997), (Yoshikura, 1975), (Jolicoeur, 1978; Jolicoeur, 1979), W097/22709.

Phage display is a powerful technique allowing the efficient screening of large numbers of random sequences in order to select the best binding sequence for any chosen application. The power of phage display is based on the ability of filamentous bacteriophages to easily express and display foreign protein moieties as fusions with phage coat proteins accompanied by the genetic material encoding that foreign protein moiety. Foreign sequences can be inserted at the amino terminus of either gene III (encoding for the minor coat protein) or gene VIII (encoding for the major coat protein) of a filamentous phage. This is usually possible without significantly interfering with the phage's life cycle (Smith, 1985),US patents 5,223,409 and 5,403,484. Phage display has been used successfully for the display of libraries of mutant immunoglobulin derived protein domains including single chain antibodies (Clackson et al., 1991; de Kruif and Logtenberg, 1996), (Stausbol-Gron et al., 1996). Phage display libraries displaying random peptides have been used extensively for the selection of epitope mapping (Jellis et al., 1993), tissue specific peptides (Heiskanen et al., 1997) and *in vivo* isolation of tissue specific peptides that bind and internalise (Pasqualini and Ruoslahti, 1996). Other molecules that have been displayed as libraries of randomised sequences are for example the HIV receptor CD4 (Roberts et al., 1996), interleukin 2 (Cabibbo et al., 1995) and plasminogen activator (Pannekoek et al., 1993). Display of these important molecules enabled the isolation of mutants with preferred and novel characteristics for application in medicine.

As described above easy and rapid procedures to isolate envelope variants would greatly facilitate the development of improved retroviral vectors including murine leukemia retroviral and lentiviral vectors. Peptide phage display has been used for the isolation of ligands that can be incorporated in a retroviral envelope to change the tropism of the original envelope and the resulting retroviral vector (Hall et al., 1997). The disadvantage of this approach is that the effect of insertion of a foreign peptide into a retroviral envelope is unpredictable and therefore the general applicability for retroviral vector improvement limited. Therefore functional display of the retroviral envelope itself is highly preferred. We disclose the functional display of a retroviral envelope on the surface of a filamentous phage. The subject of this invention opens up a number of possibilities for the development of improved retroviral, non-viral and phage vectors including lentiviral vectors by making libraries of displayed retroviral envelope mutants and selecting appropriate mutant envelopes by *in vitro, in situ* or *in vivo* screening.

In conclusion, the concept of the use of retroviruses in human gene therapy is well documented (Gordon and Anderson, 1994; Havenga et al., 1997; Vile et al., 1996). However, it would be clearly advantageous and desirable to devise a strategy for targeted delivery of retroviruses, and modification of the infection spectrum by using phage display of libraries of functional retroviral envelopes.
In the present invention we describe the methodology and materials to rapidly and efficiently generate mutant retroviral envelope molecules including but not limited to ecotropic murine leukemia retrovirus envelope proteins. These methods and materials are extremely useful for the fields of gene therapy and virology.

The present invention discloses examples of methods to develop novel retroviral envelope sequences that are optimised for desired target cells including but not limited to hemopoietic stem cells. We have developed retroviral envelope display using a filamentous phage. We have used the ecotropic Moloney murine leukemia retroviral envelope sequence as a model retroviral envelope molecule. Five different constructs were made encompassing various parts of the gp70 receptor binding moiety of the envelope. In all 5 constructs the retroviral envelope signal peptide was replaced by the pelB leader sequence of m13 gpIII. All constructs displayed gpIII/envelope fusion protein as addressed by Western blotting of envelope phages. Those constructs encompassing variable regions A and B plus or minus the polyproline hinge region were found to efficiently bind to mCAT1 expressing human 911 cells and not to control cells and thus displayed functional gp70. The construct displaying all of gp70 did exhibit functionality but to a lesser extent. Finally, constructs that encode only variable region A and B or only A did not show binding to mCAT1 cells. Infection of mCAT1 expressing human cells with ecotropic retrovirus decreased the binding of the envelope displaying phages confirming specific binding of the envelope displaying phages to the ecotropic retroviral receptor. This invention opens up a number of possibilities for the development of improved retroviral vectors including lentiviral vectors by making libraries of displayed envelope mutants and selecting appropriate mutant envelopes by *in vitro, in situ* or *in vivo* screening.

The methodology uses filamentous phage display of retroviral envelope molecules or parts thereof that can be used to develop gene transfer vehicles such as retroviral, lentiviral, phage gene transfer or non-viral gene transfer vehicles.

Included in the present invention are filamentous phages displaying mutant envelope molecules that can be used to transfer genes into cells by modification of the phage genome using techniques known in the art. This would combine the ease of producing bacteriophages with the highly efficient binding and entry of mammalian cells by mutant retrovirus envelopes.

Selection for protein mutants with preferred characteristics from envelope libraries disclosed in this application can be done using *in vitro* or *in vivo* selection methods or a combination of both. Phage libraries displaying mutant envelopes can be subjected to a number of selection rounds on desired target cells or tissues by injecting the libraries intravenously into the bloodstream of animals including but not limited to mice *in vivo* combined with a selection on relevant human tissues.
By way of illustration and not intended to be a limitation human chimeric NOD-SCID mice (Dick et al., 1997) which have a functional human hemopoiesis can be used to inject envelope display libraries followed by sampling the bone marrow and isolation of the targeted phages after various periods of time. This is then followed by amplification of the envelope displaying phages in E.coli and repeated *in vivo* selections. The *in vivo* selections can be combined with selections on purified samples of human hemopoietic stem cells including but not limited to CD34^{positive} CD38 ^{negative} or
CD34^{positive} (CD33, CD38, CD71)^{negative} cells isolated from human bone marrow, cord blood or growth factor mobilised peripheral blood using flow cytometry (Knaan-Shanzer et al., 1996).

Preferred are mutant retroviral envelopes that are derived from wild-type envelopes including but not limited to ecotropic, amphotropic and GALV retroviral envelopes by employing the disclosed envelope display technology and which employ a novel or better receptor molecule to enter the target cell by binding or improved binding to this novel or native receptor or receptors. These new retroviral envelope molecules, when incorporated in a retroviral virion, will be able to infect the receptor positive cells such as human PHSCs with increased specificity and efficiency. Using the methods described herein other retroviral envelope molecules can be displayed and converted into libraries of mutant envelopes including but not limited to GALV, amphotropic envelope and spleen necrosis viral envelope (Battini et al., 1992),(Dornburg, 1995). The mutant retroviral envelopes can be used to pseudotype recombinant type C retrovirus including but not limited to murine leukemia retroviral vectors. In a further embodiment of the present invention these new mutant envelopes can be used to pseudotype lentiviral vectors including equine or HIV derived lentiviral vectors (Kafri et al., 1997; Kim et al., 1998; Poeschla et al., 1996; Rizvi and Panganiban, 1992),(Miyoshi et al., 1997; Naldini et al., 1996b).

In a further embodiment of the present invention envelopes with ligands inserted such as a growth factor or a synthetic peptide and which function in a retrovirion but insufficient to be useful can be displayed using the technology disclosed in this invention. Display of libraries of randomised or mutagenized versions of these ligand containing envelopes will enable the selection of improved variants while retaining the ligand mediated specificity. An example of such a envelope is disclosed in W097/05266 and is an ecotropic envelope containing an immunoglobulin binding domain of a Fc receptor.

In a further embodiment of this invention retroviral envelope display can be used to evolve retroviral envelopes *in vivo* and *in vitro* to envelopes that are less sensitive i.e. resistant to the host's immune system. This application enables further improvement and applicability of retroviral vectors.

Any mutant envelope molecules or parts thereof made according to the methods described herein can then be ligated into full length mammalian retroviral envelope expression constructs and introduced in cell lines expressing and containing all the sequences necessary for the generation of infectious and functional retroviral particles including but not limited to cell lines that express murine leukemia gag-pol constructs and a retroviral vector containing long terminal repeats (LTRs),and retroviral RNA packaging signals such as those vectors described in WO96/35798. The mutant envelopes made according to the subject material of this invention can also be used to pseudotype vectors other than murine leukemia retroviral vectors including but not limited to lentiviral vectors (Naldini et al., 1996a).
Another embodiment of this invention is to use the disclosed retroviral envelope display technology to develop retroviral envelope mutants derived from pathogenic retroviruses and which can be used to block productive infection of cells in a human patient. Mutant envelope displaying phages that block a receptor can then be used as such or recombinant soluble mutant protein can be made thereof and used to treat pathogenic virus infections.

Another embodiment of this invention is to employ the envelope mutants made according tot the methods described herein in non-viral gene transfer vehicles inlcuding but not limited to incorporation in liposomes and phosphazenes (WO97/07226). This can be done by producing mutant envelopes as recombinant proteins or as envelope displaying phages and mixing or chemically linking them with liposomal or phosphazene preparations carrying a DNA expression construct of a therapeutic DNA sequence. Alternatively the retroviral envelope displaying phages itself may be used as the vector for the therapeutic sequence and modified to a DNA vector.

The skilled artisan will be able to apply the teaching of the present invention to other virus capsid or envelope or non-viral gene transfer molecules or vehicles than those exemplified herein without departing from the present invention and therefore the examples presented are illustrations and not limitations. It is intended that all such other examples be included within the scope of the appended claims.

### Example 1. Construction and generation of phagemids displaying ecotropic retroviral envelope

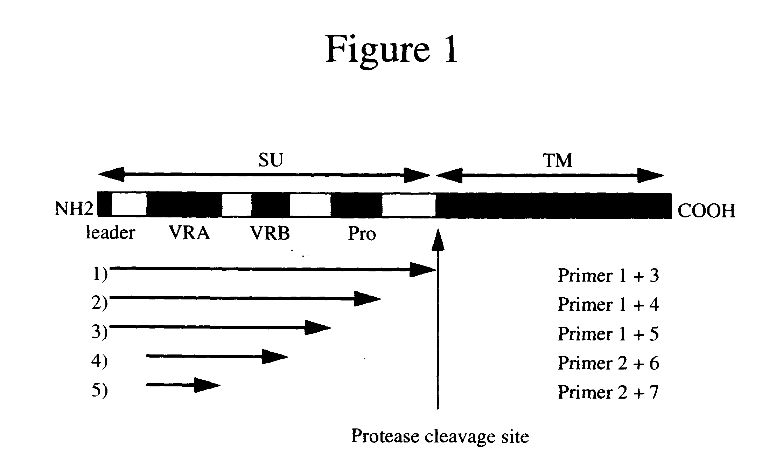

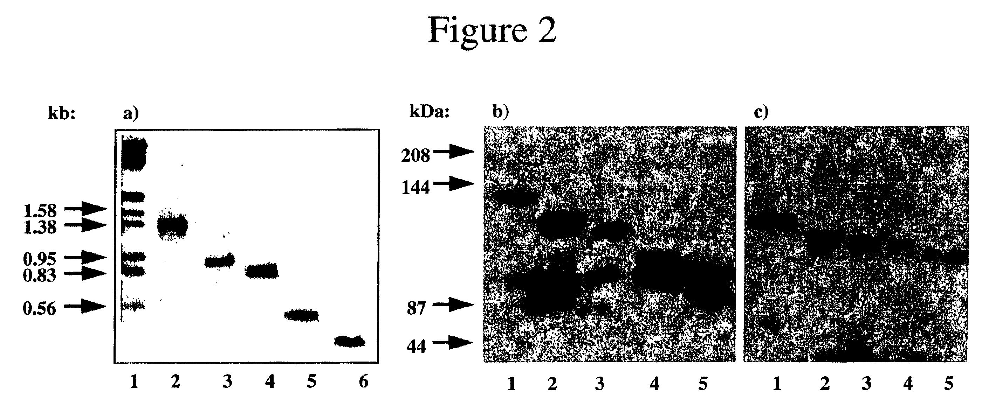

The SurfZAPÔ Vector system (Stratagene) was used for the construction of ecotropic retroviral envelope displaying phagemids. The system is based on a bacteriophage lambda vector in which the inserted sequences are displayed as N-terminal fusion proteins with the minor coat protein pIII (gpIII). Five different ecotropic envelope fragments varying in length (See figure 1) were amplified by PCR from the construct pMLV-K which contains a complete ecotropic retroviral genome (Moloney) cloned into pBR322 (Miller and Verma, 1984). The primers that were used for the construction of envelope display phagemids are summarised in table 1. The resulting constructs are designated gpIII/env1, gpIII/env2, gpIII/env3, gpIII/env4 and gpIII/env5. The PCR reaction was performed with 20 picomoles sense primer, 20 picomoles antisense primer, 1 nanomoles dNTP mixture, 1 nanogram pMLV-K, 1x buffer B (2 mM MgSO₄, 5x buffer provided with the enzyme) and 1 unit of ELONGASEÔ Enzyme mix (Life Technologies), adjusted to 50 ml with H₂O and 25 ml mineral oil on top. The following program was used on a Biometra TRIO-Thermoblock: 30 sec 94°C, 30 cycles of 30 sec 94°C, 30 sec 55°C (construct 1 and 4) or 60°C (construct 5) or 68°C (construct 2 and 3) and 30 sec 68°C, ending the cycles with a single step of 10 min 72°C. The PCR fragments were purified using Geneclean DNA purification kit followed by digestion with NotI and SpeI and cloned into the SurfZAP vector arms (NotI (left arm), SpeI (right arm)). This was followed by *in vitro* packaging of the constructs using Stratagene's Gigapack® Gold packaging extracts and amplification of the resulting lambda phage in XL1-blue *E. coli* bacteria. The lambda phages were converted to pSurfscript SK(-) phagemids by co-infection of XL1-blue bacteria with lambda phages and ExAssist helper phage. After amplification of the excised phagemids displaying phages were prepared as described in example 2. For further details the reader is referred to the instruction manual of the SurfZAP vector system (Stratagene, cat# 240211 and 240611). Expression of the gpIII/ envelope fusion proteins was tested by Western blot analysis with lysates made from bacteria and phage. To prepare the bacterial lysates Luria Broth medium containing 2% glucose and 100 mg/ml ampicillin was inoculated from a glycerol stock containing the gpIII/env constructs in SOLR bacteria. After 14-16 hours of shaking (225 rpm) at 37°C 1 ml of the culture was spun for 30 sec at 14000 rpm at 4°C (Eppendorf centrifuge #5402). The bacterial pellet was washed in 0.5 ml icecold 1M Tris-HCl pH 7.5 followed by resuspension in 200 ml H₂O and 100 ml 3x Laemmli-buffer. After 20 sec of vortexing the suspension was incubated for 5 min at 95°C and subsequently sonicated for 30 sec with an amplitude of 10 microns (Sonyprep 150). After centrifugation for 10 min at 14000 rpm, 4°C the lysates were transferred to fresh tubes and tested by Western blot analysis (20 ml per sample). The phage lysates were prepared by mixing 100 ml phages (prepared as described in the next paragraph) with 50 ml 3x Laemmli-buffer followed by incubation for 5 min at 95°C. For the Western blot analysis 25 ml per sample was used.
The five ecotropic retroviral envelope fragments for phagemid construction were obtained from the construct pMLV-K by PCR (see figure 2 panel a), digested with NotI and SpeI and ligated into the SurfZAP vector. The gpIII/envelope constructs were tested by restriction analysis and sequencing of the DNA inserts (BaseClear, Leiden) and found to be correct. All constructs had the original signal peptide of the viral sequence replaced by an E.coli/m13 signal peptide, the pelB leader sequence. Furthermore carboxy terminal linking of the envelope sequence to the pIII protein was done through a GGGGS linker sequence (see figure 1, table 1 and 2). The various constructs differ in the sequences amino or carboxy terminal of the variable regions A and B, the regions involved in receptor recognition. Construct 1 contains the full gp70 sequence until the protease cleavage site, including the variable regions A and B and the polyproline hinge region (amino acid residues 34-468). Construct 2 is like construct 1 but stops just after the polyproline region (amino acid residues 34-308). Construct 3 is like constructs 1 or 2 but stops before the beginning of the polyproline region (amino acid residues 34-266). Construct 4 encompasses the variable region A and B (amino acid residues 83-213). and construct 5 only region A the key receptor binding domain (amino acid residues 83-163). Numbering of amino acid sequences was done according to the unprocessed envelope sequence as deposited in the Swiss prot database with accession number P03385 and starting from the viral signal peptide. For further details see table 2.

Display of the fusion proteins was analysed using bacterial and phage lysates by SDS-PAGE and Western blotting using a monoclonal antibody against the gene III protein of m13. The results of such a Western blotting experiment are shown in figure 2. The bacterial lysates show bands corresponding to the expected length of the open reading frames of the DNA constructs (figure 2 panel a and figure 1) suggesting that the fusion proteins are at least expressed correctly and are stable in the *E.coli* host. Display of the envelope fusion proteins was studied by performing a Western analysis similar as for the corresponding bacterial lysates but with approximately 6 x 10⁹ cfus per lane of lysed phage preparations. The results of this analysis indeed show expression of envelope fusion proteins. Furthermore the expression patterns for the five constructs reveal in size descending protein bands in line with the descending pattern of the DNA constructs.

### Example 2 binding of displayed ecotropic envelope to it's natural receptor mCAT1

To study mCAT1 receptor binding of the envelope displaying phages both 911-pcDNA3 and 911-mCAT1 cell lines were used. For making these two cell lines the human E1 immortalised cell line 911 (Fallaux et al., 1996) was transfected either with the mCAT1 expression construct pcDNA3-mATRC1 (Albritton et al., 1993; Albritton et al., 1989) or with the pcDNA3 vector itself. The transfection was followed by selection for neomycine resistance in 1 mg/ml Geneticin (Life technologies) and by cloning the 911-mCAT1 cells. A cloned cell line designated 911-mCAT1-3H7 was isolated which showed high expression of mCAT1 mRNA as determined by Northern blot analysis. Functionality of the ecotropic retroviral receptor mCAT1 in 911 cells was shown by infection of these cells with a ecotropic retroviral vector and subsequent measurement of the marker gene. Control 911-pcDNA3 cells could not be transduced and 911-mCAT1 cells could be transduced as effectively as murine 3T3 cells. Both cell lines were maintained in Dulbecco's modified eagle medium (DMEM) (Life technologies) supplemented with 10% foetal bovine serum (FBS).

To prepare displaying phages Luria Broth medium containing 2 % glucose and 100 mg/ml ampicillin was inoculated from a glycerolstock containing the gpIII/env constructs in SOLR bacteria. After 14-16 hours shaking (225 rpm) at 37 °C the culture was diluted to OD₆₀₀=0.05 and incubated for 1.5-2 hours at 37 °C with shaking until OD₆₀₀=0.5. Infection with helper phage was performed by adding 1 x10¹⁰ pfu VCSM13 (Stratagene) per ml culture and incubating at 37 °C for 30 min. Afterwards the cells were spun down for 10 min at 4000 rpm (Heraeus, Minifuge RF), resuspended in LB, 50 mg/ml kanamycin and 100 mg/ml ampicillin and incubated for 14-16 hours at 37 °C with shaking. Phage precipitation was carried out by spinning the cells for 20 min at 4000 rpm, adding 1/5 volume 20 % Polyethylene Glycol 8000 (PEG), 2.5 M NaCl to the supernatant and incubating 1 hour on ice. The phages were spun down for 20 min at 4000 rpm and resuspended in PBS. Residual bacteria were spun down for 2 min at 14000 rpm at 4 °C (Eppendorf centrifuge #5402). The supernatant was precipitated by PEG for the second time by adding 1/5 volume 20 % PEG, 2.5 M NaCl and incubating on ice for 20 min followed by centrifugation for 20 min at 4000 rpm at 4 °C and the pellets were resuspended in PBS. Residual bacteria were spun down for 2 min at 14000 rpm at 4 °C. Phage supernatants were stored at 4 °C until further use. The titer was determined by adding an equal amount of XL1-blue bacteria in 10 mM MgSO₄, OD₆₀₀=0.5 to several dilutions of the phage preparations and plating this mix on LB plates containing 2 % glucose and 100 mg/ml ampicillin. After 14-16 hours incubation at 37 °C the titers were determined using the following formula: number of colonies x dilution factor x 1000/ volume plated = cfu/ml.

In a 15 ml polypropylene tube 0.5-1 x10⁶ cells were preincubated in 3 ml PBS containing 10% FBS and 2% non-fat milkpowder (Brand name Elk) for 30 min at room temperature on a slow turning rotator. At the same time 1 x10¹⁰ - 1x10¹¹ envelope displaying phages were preincubated in 3 ml of PBS, 10% FBS and 2% non-fat milkpowder (Brand name: Elk) for 30 min at room temperature on a slow turning rotator. Subsequently, the cells were spun down for 5 min at 1500 rpm (Heraeus, Minifuge RF) and resuspended in the preincubated phage solution. After 1 hour of rotation at room temperature, the cells with the bound phage were washed 10 times with PBS, 10% FBS and 2% non-fat milkpowder (Elk) and twice with PBS. Then the cell pellet was resuspended in H₂O and an equal volume of 200 mM Triethylamine (Sigma) was added dropwise while vortexing. After 5 min of incubation at room temperature, the suspension was neutralised by dropwise addition of Tris-HCl pH 7.5 (final concentration 0.33 M) while vortexing. The cells were spun down for 5 min at 14000 rpm (Eppendorf centrifuge) and supernatant containing the phage was transferred to a new tube. The phages were stored at 4°C and titered as described in example 1 and 2.

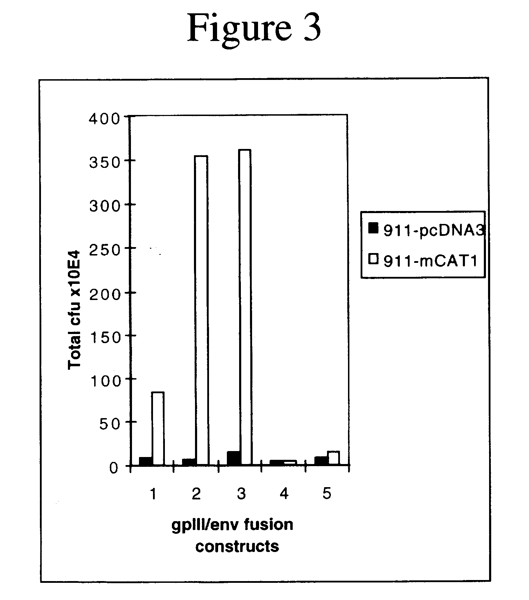

Following the protocol for preparation of gpIII/envelope displaying the titer of the phage batches varied from 2.7 x10¹⁰ to 1 x10¹² colony forming units (cfu) per ml (see table 3). Therefore display of the gpIII/ envelope fusion proteins does not strongly interfer with the production of phage particles. We now wanted to know whether the displayed envelope moieties in fact are functional and behave as retroviral gp70 and thus bind to the mCAT1 receptor for ecotropic retrovirus. For this purpose we carried out binding experiments using human cells that express the mCAT1 receptor protein. For these binding experiments 1 x10¹⁰ -1 x10¹¹ phages displaying ecotropic retroviral envelope protein or parts thereof were incubated with 0.5-1 x10⁶ 911 cells stably expressing the mCAT1 receptor or 911 cells stably transfected with the pcDNA3 vector. The number of envelope fusion phages eluted from control cell line 911-pcDNA3 averaged around 1 x10⁴ cfus. On 911-mCAT1 cells different elution behaviour was visible, from 2.3 x10⁴ to 3.6 x10⁶ cfus. For gpIII/env 4 and gpIII/env 5 the number of eluted phages after selection on 911-mCAT1 cells was comparable to the number of eluted phages after selection on 911-pcDNA3 cells. Phages of the gpIII/env 1, gpIII/env 2 and gpIII/env 3 constructs show respectivily 11, 97 and 26 times more eluted phage from 911-mCAT1 cell line as compared to the 911-pcDNA3 cell line (figure 3). Furthermore the stability of envelope displaying phages (gpIII/env3) was addressed by measuring binding to mCAT1 and titer on bacteria at various periods of time after preparation. After 1 day, 2 weeks and 4 weeks after preparation no significant differences were observed. These results show that 3 of the 5 constructs clearly display functional ecotropic envelope. These phages can thus be used to make libraries of mutants that can be used to develop preferred envelopes for applications in gene therapy, molecular biology and virology.

### Example 3. Inhibition of envelope phage binding by ecotropic retroviral particles

Functional phage display of ecotropic envelope also implies that mCAT1 expressing cells infected or preincubated with ecotropic retrovirus should decrease the binding of the envelope displaying phages to mCAT1. To confirm the latter both control and mCAT1 expressing cells were preincubated with replication competent ecotropic retrovirus produced on murine fibroblasts (3T3-RCR).This was done as follows: Two days before the 911-pcDNA3 and 911-mCAT1 cells were used for the binding experiment they were seeded in a T25 cm² flask with DMEM containing 10% FBS and 4 mg/ml polybrene (Hexadimethrine bromide, Sigma). After 12-16 hours incubation at 37 °C and 10 % CO₂ the medium was discarded and replaced with 4 ml replication competent ecotropic retrovirus produced on murine fibroblasts (NIH 3T3) or 4 ml DMEM containing 10% FBS (control). After infection of 12-16 hours at 37 °C and 10 % CO₂ the cells were harvested by trypsinisation and used in envelope phage binding experiments.

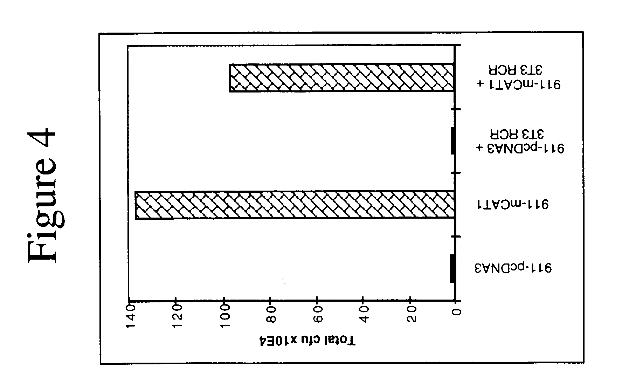

Using this protocol the number of eluted gpIII/env 3 phages from mCAT1 expressing 911 cells was decreased on average 35 % (figure 4). This further confirms that the phages displaying ecotropic envelope indeed have features similar to the gp70 moiety displayed on a native ecotropic retroviral virion.

### Example 4 Generation of an envelope display library encompassing mutant envelope sequences

The plasmid DNA constructs gpIII/env2 and gpIII/env3 in pSURFscript were used to construct two libraries of randomised, mutant envelope sequences. Two different techniques to generate pools of mutants can be followed:
(1) The gpIII/env2 plasmid DNA was passed through the mutagenic E.coli strain XL1-Red (genotype: endA1 gyrA96 thi-1 hsdR17 supE44 relA1 lac mutD5 mutS mutT Tn10 (Tetr)). The XL1-Red strain is deficient in three of the primary DNA repair pathways in E.coli, the mutS, mutD and mutT, making its mutation rate approximately 5,000-fold higher than that of its wild-type parent (Greener et al., 1996; Greener et al., 1997). GpIII/env2 or gpIII/env3 plasmid DNA was used to transform competent XL1-Red cells that were purchased from Stratagene (catalogue no: 200129) with a transformation efficiency of approximately 10⁶ cfus/mg pUC18 DNA. Transformed cells were plated on large LB-agar petri-dishes with ampicillin (100 mg/ml) and incubated at 37 °C overnight. Ampicillin resistant colonies were scraped off the plates and used to inoculate an 1000 ml Erlenmeyer flask with 250 ml LB and ampicillin (100 mg/ml). The Erlenmeyer flasks were transferred to a shaking incubator set at 37 °C and incubated overnight or as long as it takes to get sufficient bacteria. The following day the cultures were subjected to a plasmid DNA isolation procedure using the Qiagen plasmid DNA column method.
   The isolated plasmid DNA is verified using Notl, SpeI and PinA1 enzyme digestions and DNA agarose gel electrophoresis.

As an example two strategies can used to generate envelope displaying libraries based on XL1-Red mutagenized gpIII/env2 and 3 DNA: (A) The XL1-Red propagated and mutagenized DNA as such was used directly to generate libraries of envelope displaying phages as described under examples 1 and 2 using XL1-Blue competent bacteria.
(B) The XL1-Red mutagenized plasmid DNA of gpIII/env2 and 3 are digested with Not1 and SpeI followed by isolation of the Notl/SpeI 869 basepairs (gpIII/env2) and 743 basepairs (gpIII/env3) fragments using DNA agarose gel electrophoresis and Qiaquick gel extraction method (Qiagen). These fragments are ligated back into gpIII/env or 3. This was done by digesting gpIII/env2 or 3 propagated in DH5alfa E.coli with NotI and SpeI and isolation of a 3631 basepair fragment. The pools of ligated molecules can then be used to generate envelope display libraries by transformation into competent XL1-Blue cells and subsequent steps as described in example 1.
(2) The second technique that was used to generate randomised envelope displaying phage libraries was shuffle PCR (Stemmer, 1994a; Stemmer, 1994b). Shuffle PCR generates libraries of mutant genes by recursive sequence recombination. This is done by digesting a PCR amplified target sequence with DNA polymerase I followed by isolation of smaller DNAs I digested fragments (30-700 basepairs). The latter is followed by Taq polymerase mediated reassembly of the full length fragment without the addition of any single strand synthetic oligonucleotides. Reassembled, shuffled target sequence is then amplified and cloned back into the appropriate vector. For the generation of a library of phages displaying randomised envelopes sequences using shuffle PCR, the following PCR was performed using pMLVK (Miller and Verma, 1984) plasmid DNA made in DH5alfa E,coli or XL1-Red E.coli bacteria as a template per 100 ml total reaction volume. For construction of a shuffled gpIII/env2 library the primer-pair ecoenv17/ecoenv12 was used and for gpIII/env3 shuffled libraries the primer-pair ecoenv17/ecoenv15 (see table 1). The PCR reactions were performed with 40 picomoles sense primer, 40 picomoles antisense primer, 2 nanomoles dNTP mixture, 1 nanogram pMLV-K or gpIII/env2 or 3, lx SuperTaq buffer and 0.5 units of SuperTaq thermostabile polymerase (both from HT Biotechnology, UK) adjusted to 100 ml with H₂O and 25 ml mineral oil on top. The following program was used on a Biometra TRIO-Thermoblock: 30 sec 94°C, 30 cycli of 30 sec 94°C, 30 sec 68°C and 30 sec 72°C, ending the cycles with a single step of 10 min at 72°C. Amplified DNA was purified using Qiaquick PCR purification cartridges and checked for appropriate size (869 basepairs for ecoenv17/ecoenv12 and 743 basepairs ecoenv17/ecoenv15) by agarose gel electrophoresis. Five micrograms of the amplified DNA was then subjected to a digestion with 0.15 units of DNAseI (Sigma D-7291)(DNAseI concentration of 0.0015 Units/ ml) in 50 mM Tris-HCl pH 7.4, 1 mM MgCl₂. Digest for 5 minutes at 25 °C, put on ice and verify extent of digestion by loading an aliquot on a 2 % agarose gel in 1 x TAE together with a lane with a suitable DNA marker to determine size range of generated fragments. If sufficient material is generated in the desired size range of 30-700 basepairs the tube is incubated for 10 minutes at 65 °C to inactivate the DNAse I enzyme. Otherwise incubate an additional 5 minutes at 25 °C and repeat the above. Once sufficient DNAse I digested material has been generated all DNAse I treated material of the two fragments can be loaded on a gel. and the fragments of 50-300 basepairs isolated from the gel using a Qiaquick gel extraction kit or equivalent. One hundred to 2000 nanograms in 100 ml final volume of fragment is then subjected to a reassembly step with a hot start of 1 minute at 94 °C and 45 cycles of 30 seconds at 94 °C, 30 seconds at 50 or 55 °C and 30 seconds at 72 °C. The composition of the buffer is identical to that used for amplification of the primary PCR products using ecoenv17/ecoenv12 and ecoenv17/ecoenv15 primer pairs except for the absence of any ecoenv primer. To monitor the extent of reassembly samples can be taken at 25,30,35, 40 and finally 45 cycles. Extent of reassembly is visualised by loading on a 1-2% agarose gel and comparison to the size of the primary PCR product, input DNAseI fragments. Reassembly is followed by amplification of reassembled, shuffled fragment to sufficient amounts of DNA for cloning back into the envelope phage display vector. For this purpose various amounts of reassembled DNA are subjected to the same PCR reactions used for generation of the primary PCR reactions and thus using the same pair of primers. Alternatively for higher yields of shuffled fragment Elongase (Life technologies) is used (see for experimental details example 1). The reassembled shuffled pool of fragments (approximately 869 basepairs in length for ecoenv17/ecoenv12 (gpIII/env2) and approximately 743 basepairs for ecoenv17/ecoenv15 (gpIII/env3)) are purified using Qiaquick PCR purification cartridges and digested with NotI and SpeI or with NotI and PinA1 followed by isolation of the digested fragments using agarose gel electrophoresis. This results in 4 different pools of fragments that can be ligated into NotI/SpeI or NotI/PinAI digested gpIII/env2 or gpIII/env3 vector fragment. The ligation mixtures are then used to generate phage libraries displaying shuffled envelope molecules as described under examples 1 and 2. Digestion with NotI and SpeI or NotI and PinA1 results in libraries displaying varying parts of the gp70 sequence as a shuffled library of mutants. The skilled artisan will be able to apply the teaching of the above to the generation of mutant envelope molecules by incubating target cells of any origin including but not limited to human CD34⁺ cells enriched for hemopoietic stem cells with the various libraries and repeating these rounds of selection until envelope displaying phages have been selected for that are optimised for binding and entry into the desired target cells. These mutant envelope molecules or parts thereof can then be ligated into full length mammalian ecotropic envelope expression constructs and introduced in cell lines expressing and containing all the sequences necessary for the generation of infectious and functional retroviral particles including but not limited to cell lines that express murine leukemia gag-pol constructs and a retroviral vector containing long terminal repeats (LTRs),and retroviral RNA packaging signals such as those vectors described in WO96/35798. The mutant envelopes made according to the subject material of this invention can also be used to pseudotype vectors other than murine leukemia retroviral vectors including but not limited to lentiviral vectors (Naldini et al., 1996a).

### Example 5

FAb phage display to select CAT1 binding antibody molecules for for example incorporation in a mutant protein.

To isolate antibodies that bind to CAT we also employed phages displaying FAb fragments encompassing the light and heavy variable and constant regions. A FAb phage display library was constructed in phage display vector pCES1 a vector derived from pCANTAB6 (McGuiness et al., 1996). The library was constructed in the filamentous E. coli phage m13 and the FAb sequences are displayed partly as N-terminal fusion proteins with the minor coat protein pIII. Two targets were used to select for peptide displaying phages which bind to the third extracellular domain of CAT. First the predicted third extracellular domain of CAT was synthesized as a synthetic peptide by Neosystem, Strassbourg, France. The N-terminus of this peptide was biotinylated and followed by three amino acid linker residues KRR, followed by the predicted sequence of the third extracellular domain. Secondly we generated cell lines that overexpress CAT as judged by steady state mRNA expression levels. A CAT expression construct which is a pcDNA3 based expression construct of CAT cDNA was employed to transfect CAT expressing cell lines followed by selection for neomycine resistance in 1 mg/ml of G418 (Geneticin, Life Technologies, Inc).

To select for FAb displaying phages that bind to the putative third extracellular domain of CAT as expressed on human cells the following selection procedure was employed. Four rounds of selection on biotinylated CAT peptide followed by three rounds of selection on CAT overexpressing cells. For selection on biotinylated CAT peptide 250 ml of FAb library (or eluted phage from the previous round) was mixed with 250 ml 4% Marvel in PBS and equilibrated while rotating at RT for 1 hour. Subsequently biotinylated CAT peptide (20-500 nM in H₂O) was added. This mix was incubated on the rotator at RT for 1 hour before 250 ml equilibrated streptavidin-dynabeads in 2% Marvel in PBS was added. After incubation on a rotator at RT for 15 min the beads with the bound phage were washed 5 times with PBS/2% Marvel/0.1% Tween, 5 times with PBS /0.1% Tween and 5 times with PBS. Then the phage were eluted by incubation with 0.1M Tri-ethyl-amine on a rotator at RT for 10 min and neutralised in 1 M Tris-HCl pH 7.4. The eluted phage were titered and amplified in TG1 before the next selection. For selection on CAT expressing cells, the cells were harvested at a confluency of about 80 % and suspended in PBS/10 % FBS/2 % Marvel to a final concentration of at least 3x10⁶ cells/ml. This cell suspension was incubated for 30 min on a rowing boat mixer (or rotator), while at the same time phage were also preincubated in PBS/10 % FBS/2 % Marvel. Then the cells were centrifuged, resuspended in the preincubated phage solution and incubated on a rowing boat mixer (or rotator) for 1 hour. Afterwards the cells were washed 10 times with PBS/10 % FBS/2 % Marvel and twice with PBS. The cells were centrifuged and resuspended in 0.6 ml water. Subsequently 0.6 ml 200 mM triethylamine was added (dropwise while vortexing). After 5 minutes the suspension was neutralised by adding 0.6 ml of 1 M Tris-HCl pH 7.4 (dropwise while vortexing).After cenrifugation (5 min, 14000 rpm) the supernatant was transferred to a new tube and titered and amplified in TG1 before the next selection. The results of these experiments show that the ratio of input over output increases upon selection on CAT peptide indicative of selection for CAT peptide binding phages. When selection on CAT positive cells was started the ratio dropped and slightly increased in the last round on CAT expressing cells.

The pools of the last 3 rounds were tested for binding to the biotinylated CAT peptide in a CAT specific ELISA and also for cell binding by flow cytometric analysis. After the last round of selection on cells the pool of FAb phages still binds to the biotinylated CAT peptide. Flow cytometric analysis showed that this pool also binds to CAT overexpressing cells. From this pool clones were analysed by fingerprint analysis. Double strand phagemid DNA was prepared and used to determine the nucleotide and deduced amino acid sequence of the displayed variable heavy and light chains by clones binding to CAT. The relevant sequences or parts thereof of this immunoglobulin can be incorporated in viral or non-viral mutant proteins that mediate binding and entry to cells and thus create gene transfer vehicles that enter cells through CAT.

### REFERENCES

Ager, S., Nilson, B.H., Morling, F.J., Peng, K.W., Cosset, F.L. and Russell, S.J. (1996) Retroviral display of antibody fragments; interdomain spacing strongly influences vector infectivity. Hum Gene Ther 7(17), 2157-64.

Albritton, L.M., Kim, J.W., Tseng, L. and Cunningham, J.M. (1993) Envelope-binding domain in the cationic amino acid transporter determines the host range of ecotropic murine retroviruses. J Virol 67(4), 2091-2096.

Albritton, L.M., Tseng, L., Scadden, D. and Cunningham, J.M. (1989) A putative murine ecotropic retrovirus receptor gene encodes a multiple membrane-spanning protein and confers susceptibility to virus infection. Cell 57(4), 659-666. Bae, Y., Kingsman, S.M. and Kingsman, A.J. (1997) Functional dissection of the Moloney murine leukemia virus envelope protein gp70. J Virol 71(3), 2092-9.

Battini, J.L., Heard, J.M. and Danos, O. (1992) Receptor choice determinants in the envelope glycoproteins of amphotropic, xenotropic, and polytropic murine leukemia viruses. J-Virol 66(3), 1468-75.

Braas, G., Searle, P.F., Slater, N.K. and Lyddiatt, A. (1996) Strategies for the isolation and purification of retroviral vectors for gene therapy. Bioseparation 6(4), 211-28.

Cabibbo, A., Sporeno, E., Toniatti, C., Altamura, S., Savino, R., Paonessa, G. and Ciliberto, G. (1995) Monovalent phage display of human interleukin (hIL)-6: selection of superbinder variants from a complex molecular repertoire in the hIL-6 D- helix. Gene 167(1-2), 41-47.

Chu, T.H., Martinez, I., Sheay, W.C. and Dornburg, R. (1994) Cell targeting with retroviral vector particles containing antibody-envelope fusion proteins. Gene Ther 1(5), 292-9 Issn: 0969-7128.

Clackson-, T., Hoogenboom, H.R., Griffiths, A.D. and Winter, G. (1991) Making antibody fragments using phage display libraries. Nature 352(6336), 624-628.

Cosset, F.L., Morling, F.J., Takeuchi, Y., Weiss, R.A., Collins, M.K. and Russell, S.J. (1995) Retroviral retargeting by envelopes expressing an N-terminal binding domain. J Virol 69(10), 6314-22 Issn: 0022-538x.

Davey, R.A., Hamson, C.A., Healey, J.J. and Cunningham, J.M. (1997) In vitro binding of purified murine ecotropic retrovirus envelope surface protein to its receptor, MCAT-1 [In Process Citation]. J Virol 71(11), 8096-102.

de Kruif, J. and Logtenberg, T. (1996) Leucine zipper dimerized bivalent and bispecific scFv antibodies from a semi-synthetic antibody phage display library. J Biol Chem 271(13), 7630-7634.

Demarquoy, J. (1993) Retroviral-mediated gene therapy for the treatment of citrullinemia. Transfer and expression of argininosuccinate synthetase in human hematopoietic cells. Experientia 49(4), 345-8.

Dick, J.E., Bhatia, M., Gan, O., Kapp, U. and Wang, J.C. (1997) Assay of human stem cells by repopulation of NOD/SCID mice. Stem Cells 15 Suppl 1, 199-203; discussion 204-7.

Dornburg, R. (1995) Reticuloendotheliosis viruses and derived vectors. Gene Ther 2(5), 301-10.

Fallaux, F.J., Kranenburg, O., Cramer, S.J., Houweling, A., Van Ormondt, H., Hoeben, R.C. and Van Der Eb, A.J. (1996) Characterization of 911: a new helper cell line for the titration and propagation of early region 1-deleted adenoviral vectors. Hum Gene Ther 7(2), 215-22.

Fass, D., Harrison, S.C. and Kim, P.S. (1996) Retrovirus envelope domain at 1.7 angstrom resolution. Nat-Struct-Biol 3(5), 465-9 issn: 1072-8368.

Gordon, E.M. and Anderson, W.F. (1994) Gene therapy using retroviral vectors. Curr Opin Biotechnol 5(6), 611-6.

Greener, A., Callahan, M. and Jerpseth, B. (1996) An efficient random mutagenesis technique using an E. coli mutator strain. Methods Mol Biol 57, 375-85.

Greener, A., Callahan, M. and Jerpseth, B. (1997) An efficient random mutagenesis technique using an E. coli mutator strain. Mol Biotechnol 7(2), 189-95.

Gunzburg, W.H. and Salmons, B. (1996) Development of retroviral vectors as safe, targeted gene delivery systems. J Mol Med 74(4), 171-82.

Hall, F.L., Gordon, E.M., Wu, L., Zhu, N.L., Skotzko, M.J., Starnes, V.A. and Anderson, W.F. (1997) Targeting retroviral vectors to vascular lesions by genetic engineering of the MoMLV gp70 envelope protein. Hum Gene Ther 8(18), 2183-92.

Havenga, M., Hoogerbrugge, P., Valerio, D. and van Es, H.H.G. (1997) Retroviral stem cell gene therapy. Stem cells 15(3), 162-179.

Heiskanen, T., Lundkvist, A., Vaheri, A. and Lankinen, H. (1997) Phage-displayed peptide targeting on the Puumala hantavirus neutralization site. J Virol 71(5), 3879-85. Hoogerbrugge, P.M., van Beusechem, V.W., Fischer, A., Debree, M., Le Deist, F., Perignon, J.L., Morgan, G., Gaspar, B., Fairbanks, L.D., Skeoch, C.H., Mosely, A., Harvey, M., Levinskey, R.J. and Valerio, D. (1996) Bone marrow gene transfer in three patients with adenosine deaminase deficiency. Gene Therapy 3, 179-183.

Januszeski, M.M., Cannon, P.M., Chen, D., Rozenberg, Y. and Anderson, W.F. (1997) Functional analysis of the cytoplasmic tail of Moloney murine leukemia virus envelope protein. J Virol 71(5), 3613-9.

Jellis, C.L., Cradick, T.J., Rennert, P., Salinas, P., Boyd, J., Amirault, T. and Gray, G.S. (1993) Defining critical residues in the epitope for a HIV-neutralizing monoclonal antibody using phage display and peptide array technologies. Gene 137(1), 63-68.

Jolicoeur, P. (1978) Genetic control of mouse leukemia virus replication. Natl Cancer Inst Monogr(48), 191-7.

Jolicoeur, P. (1979) The Fv-1 gene of the mouse and its control of murine leukemia virus replication. Curr Top Microbiol Immunol 86, 67-122.

Kafri, T., Blomer, U., Peterson, D.A., Gage, F.H. and Verma, I.M. (1997) Sustained expression of genes delivered directly into liver and muscle by lentiviral vectors. Nat Genet 17(3), 314-7.

Kaptein, L.C., Greijer, A.E., Valerio, D. and van Beusechem, V.W. (1997) Optimized conditions for the production of recombinant amphotropic retroviral vector preparations. Gene Ther 4(2), 172-6.

Kavanaugh, M.P., Miller, D.G., Zhang, W., Law, W., Kozak, S.L., Kabat, D. and Miller, A.D. (1994) Cell-surface receptors for gibbon ape leukemia virus and amphotropic murine retrovirus are inducible sodium-dependent phosphate symporters. Proc-Natl-Acad-Sci-U-S-A 91(15), 7071-5 issn: 0027-8424.

Kim, J.W., Closs, E.I., Albritton, L.M. and Cunningham, J.M. (1991) Transport of cationic amino acids by the mouse ecotropic retrovirus receptor. Nature 352(6337), 725-728.

Kim, V.N., Mitrophanous, K., Kingsman, S.M. and Kingsman, A.J. (1998) Minimal requirement for a lentivirus vector based on human immunodeficiency virus type 1. J Virol 72(1), 811-6.

Knaan-Shanzer, S., Valerio, D. and van Beusechem, V.W. (1996) Cell cycle state, response to hemopoietic growth factors and retroviral vector-mediated transduction of human hemopoietic stem cells. Gene Ther 3(4), 323-333.

MacKrell, A.J., Soong, N.W., Curtis, C.M. and Anderson, W.F. (1996) Identification of a subdomain in the Moloney murine leukemia virus envelope protein involved in receptor binding. J-Virol 70(3), 1768-74.

Marin, M., Noel, D., Valsesia-Wittman, S., Brockly, F., Etienne-Julan, M., Russell, S., Cosset, F.L. and Piechaczyk, M. (1996) Targeted infection of human cells via major histocompatibility complex class I molecules by Moloney murine leukemia virus-derived viruses displaying single-chain antibody fragment-envelope fusion proteins. J Virol 70(5), 2957-2962.

Masuda, M., Hanson, C.A., Alvord, W.G., Hoffman, P.M., Ruscetti, S.K. and Masuda, M. (1996a) Effects of subtle changes in the SU protein of ecotropic murine leukemia virus on its brain capillary endothelial cell tropism and interference properties. Virology 215(2), 142-51 Issn: 0042-6822.

Masuda, M., Masuda, M., Hanson, C.A., Hoffman, P.M. and Ruscetti, S.K. (1996b) Analysis of the unique hamster cell tropism of ecotropic murine leukemia virus PVC-211. J Virol 70(12), 8534-8539.

Miller, A.D. and Verma, I.M. (1984) Two base changes restore infectivity to a noninfectious molecular clone of Moloney murine leukemia virus (pMLV-1). J Virol 49(1), 214-222.

Miller, D.G. and Miller, A.D. (1994) A family of retroviruses that utilize related phosphate transporters for cell entry. J-Virol 68(12), 8270-6 issn: 0022-538x.

Miyoshi, H., Takahashi, M., Gage, F.H. and Verma, I.M. (1997) Stable and efficient gene transfer into the retina using an HIV-based lentiviral vector. Proc Natl Acad Sci U S A 94(19), 10319-23.

Naldini, L., Blomer, U., Gage, F.H., Trono, D. and Verma, I.M. (1996a) Efficient transfer, integration, and sustained long-term expression of the transgene in adult rat brains injected with a lentiviral vector. Proc Natl Acad Sci U S A 93(21), 11382-8.

Naldini, L., Blomer, U., Gallay, P., Ory, D., Mulligan, R., Gage, F.H., Verma, I.M. and Trono, D. (1996b) In vivo gene delivery and stable transduction of nondividing cells by a lentiviral vector [see comments]. Science 272(5259), 263-7.

Orlic, D., Girard, L.J., Jordan, C.T., Anderson, S.M., Cline, A.P. and Bodine, D.M. (1996) The level of mRNA encoding the amphotropic retrovirus receptor in mouse and human hematopoietic stem cells is low and correlates with the efficiency of retrovirus transduction. Proc Natl Acad Sci U S A 93(20), 11097-11102.

Pannekoek, H., van Meijer, M., Schleef, R.R., Loskutoff, D.J. and Barbas, C.F.d. (1993) Functional display of human plasminogen-activator inhibitor 1 (PAI-1) on phages: novel perspectives for structure-function analysis by error- prone DNA synthesis. Gene 128(1), 135-140.

Pasqualini, R. and Ruoslahti, E. (1996) Organ targeting in vivo using phage display peptide libraries. Nature 380(6572), 364-366.

Poeschla, E., Corbeau, P. and Wong-Staal, F. (1996) Development of HIV vectors for anti-HIV gene therapy. Proc Natl Acad Sci U S A 93(21), 11395-9.

Rizvi, T.A. and Panganiban, A.T. (1992) Simian immunodeficiency virus vectors: replication and pseudotyping. J Med Primatol 21(2-3), 69-73.

Roberts, B.L., Markland, W. and Ladner, R.C. (1996) Affinity maturation of proteins displayed on surface of M13 bacteriophage as major coat protein fusions. Methods Enzymol 267, 68-82.

Skov, H. and Andersen, K.B. (1993) Mutational analysis of Moloney murine leukaemia virus surface protein gp70. J-Gen-Virol 74(Pt 4), 707-14.

Smith, G.P. (1985) Filamentous fusion phage: novel expression vectors that display cloned antigens on the virion surface. Science 228(4705), 1315-1317.

Stausbol-Gron, B., Wind, T., Kjaer, S., Kahns, L., Hansen, N.J., Kristensen, P. and Clark, B.F. (1996) A model phage display subtraction method with potential for analysis of differential gene expression. FEBS Lett 391(1-2), 71-75.

Stemmer, W.P. (1994a) DNA shuffling by random fragmentation and reassembly: in vitro recombination for molecular evolution. Proc-Natl-Acad-Sci-U-S-A 91(22), 10747-51 issn: 0027-8424.

Stemmer, W.P. (1994b) Rapid evolution of a protein in vitro by DNA shuffling. Nature 370(6488), 389-91 issn: 0028-0836. Takeuchi, Y., Cosset, F.L., Lachmann, P.J., Okada, H., Weiss,

R.A. and Collins, M.K. (1994) Type C retrovirus inactivation by human complement is determined by both the viral genome and the producer cell. J Virol 68(12), 8001-7.

Taplitz, R.A. and Coffin, J.M. (1997) Selection of an avian retrovirus mutant with extended receptor usage. J Virol 71(10), 7814-9.

Thomas, A., Gray, K.D. and Roth, M.J. (1997) Analysis of mutations within the cytoplasmic domain of the Moloney murine leukemia virus transmembrane protein. Virology 227(2), 305-13.

Van Beusechem, V.W., Bakx, T.A., Kaptein, L.C., Bart-Baumeister, J.A., Kukler, A., Braakman, E. and Valerio, D. (1993) Retrovirus-mediated gene transfer into rhesus monkey hematopoietic stem cells: the effect of viral titers on transduction efficiency. Hum Gene Ther 4(3), 239-47.

van Beusechem, V.W., Kukler, A., Heidt, P.J. and Valerio, D. (1992) Long-term expression of human adenosine deaminase in rhesus monkeys transplanted with retrovirus-infected bone-marrow cells. Proc Natl Acad Sci U S A 89(16), 7640-4.

van Es, H.H.G., Knaan, S., Camphorst, S., Verlinden, S. and Valerio, D. (1996) Expression studies of the amphotropic receptor GLVR2 in mammalian cells and tissues including human CD34+ cells. Cold Spring Harbor Gene therapy meeting, Abstract 309.

van Zeijl, M., Johann, S.V., Closs, E., Cunningham, J., Eddy, R., Shows, T.B. and O'Hara, B. (1994) A human amphotropic retrovirus receptor is a second member of the gibbon ape leukemia virus receptor family. Proc-Natl-Acad-Sci-U-S-A 91(3), 1168-72 issn: 0027-8424.

Vile, R.G., Tuszynski, A. and Castleden, S. (1996) Retroviral vectors. From laboratory tools to molecular medicine. Mol Biotechnol 5(2), 139-58.

von Kalle, C., Kiem, H.P., Goehle, S., Darovsky, B., Heimfeld, S., Torok Storb, B., Storb, R. and Schuening, F.G. (1994) Increased gene transfer into human hematopoietic progenitor cells by extended in vitro exposure to a pseudotyped retroviral vector. Blood 84(9), 2890-7 issn: 0006-4971.

Weiss, R.A. (1996) Retrovirus classification and cell interactions. J Antimicrob Chemother 37 Suppl B, 1-11.

Wilson, C.A., Farrell, K.B. and Eiden, M.V. (1994) Properties of a unique form of the murine amphotropic leukemia virus receptor expressed on hamster cells. J-Virol 68(12), 7697-703 issn: 0022-538x.

Yoshikura, H. (1975) Adaptation of N-tropic Friend leukaemia virus and its murine sarcoma virus pseudotype of non-permissive B-type C57BL/6 mouse cell line. J Gen Virol 29(1), 1-9.

Zhao, Y., Lee, S. and Anderson, W.F. (1997) Functional interactions between monomers of the retroviral envelope protein complex [In Process Citation]. J Virol 71(9), 6967-72.

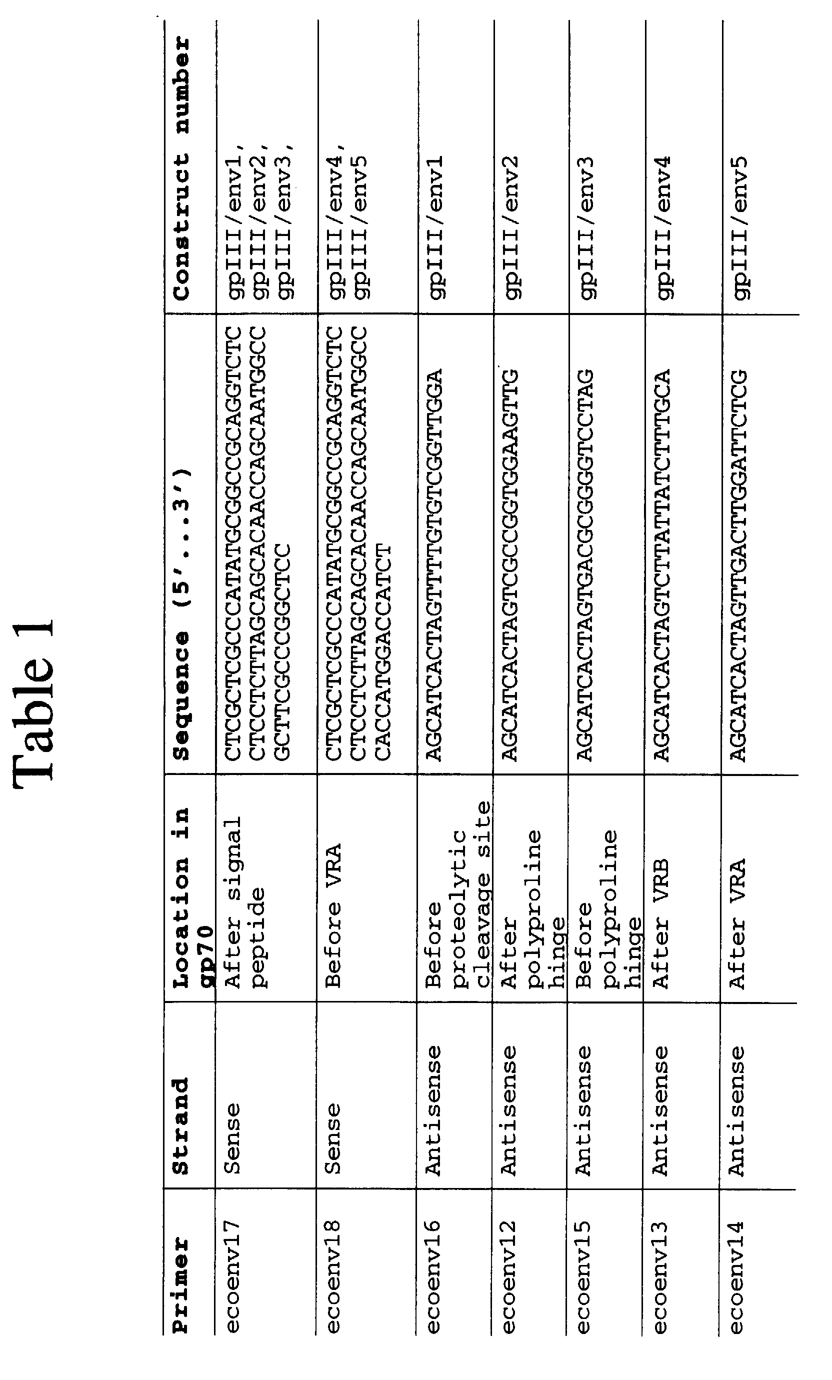

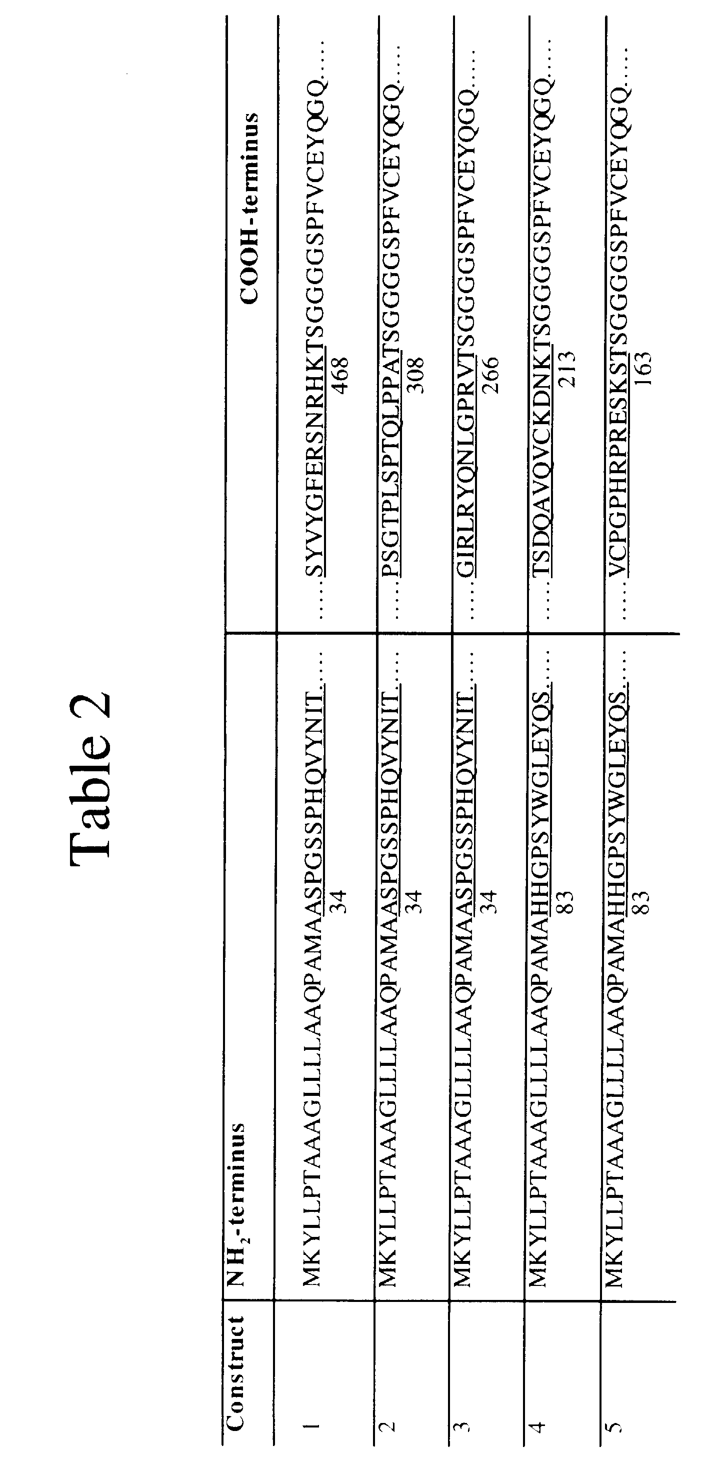

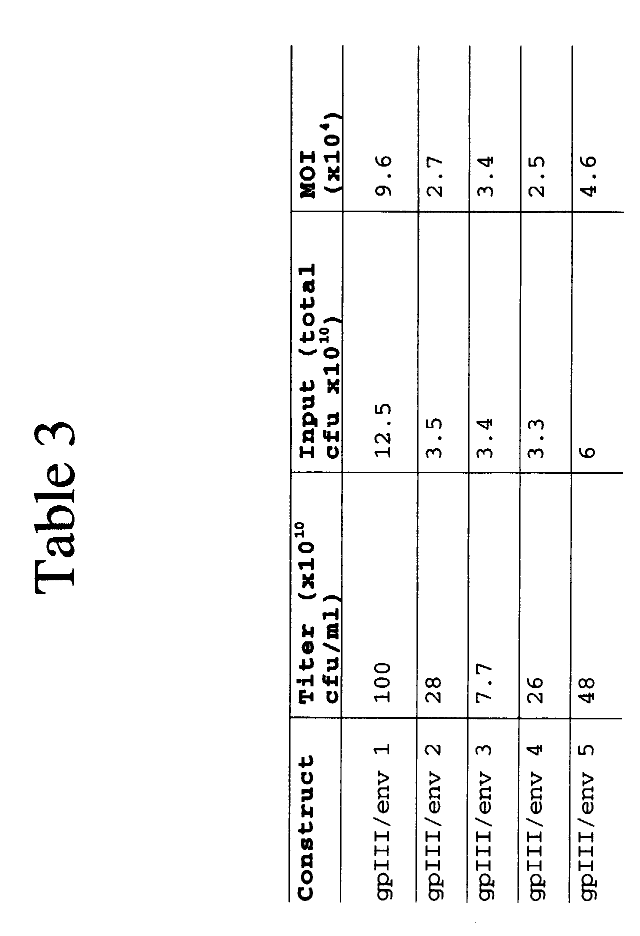

## Claims

1. A method for selecting at least one mutant protein derived from a viral protein as a ligand capable of binding to a cell-surface receptor, comprising displaying at least one mutant of said protein on the surface of a micro-organism expressing said protein, further comprising selecting at least one such a mutant micro-organism for its capacity to bind to said receptor.

2. A method according to claim 1 further comprising incorporating said protein in a virus-like particle or gene-delivery vehicle allowing targeting of said virus-like particle or gene-delivery vehicle to cells carrying said receptor.

3. A method according to claim 1 or 2 wherein said micro-organism is a filamentous phage.

4. A method according to any of claims 1 to 3 wherein said protein comprises at least a fragment of an envelope protein, preferably derived from a retrovirus.

5. A method according to any of claims 1 to 3 wherein said protein comprises at least a fragment of a capsid protein, preferably derived from an adenovirus.

6. A method according to any of claims 1 to 3 wherein said protein comprises a at least a fragment of a coat protein, preferably derived from a bacteriophage.

7. A method according to any one of claims 1 to 6 wherein said cell-surface receptor is an amino acid transporter, preferably a cationic amino acid transporter such as mCAT1.

8. A method according to claim 7 wherein said mutant protein is derived from glycoprotein 70 (gp70) of Moloney murine leukemia virus.

9. A virus-like particle or gene-delivery vehicle obtainable by a method according to any one claims 1 to 8.

10. A virus-like particle or gene-delivery vehicle according to claim 9 for use as a gene-transfer vehicle.

11. A method for selecting a filamentous phage expressing a protein capable of binding to a ligand comprising constructing a phage library, enriching said library for phages having desired binding characteristics by at least one round of selection of phages for their capacity to bind to a synthetic peptide derived from said ligand, further comprising enriching said library for phages having desired binding characteristics by at least one round of selection of phages for their capacity to bind to a cell expressing said ligand.

12. A method according to claim 9 wherein said ligand is a amino acid transporter, preferably mCAT1.


### Annex to the description

SEQUENCE LISTING

<110> Introgene BV

<120> Display of viral proteins

<130> P22398EP01

<140> EP 99201592.5
<141> 1999-05-20

<150> EP 98201678.4
<151> 1998-05-20

<160> 15

<170> PatentIn Ver. 2.1

<210> 1
<211> 5
<212> PRT
<213> Moloney murine leukemia virus

<220>
<221> PEPTIDE
<222> (1)..(5)
<223> /Note="linker sequence"

<400> 1
Gly Gly Gly Gly Ser
  1               5

<210> 2
<211> 75
<212> DNA
<213> Moloney murine leukemia virus

<220>
<221> primer_bind
<222> (1)..(75)
<223> /note="primer ecoenv17"

<400> 2
ctcgctcgcc catatgcggc cgcaggtctc ctcctcttag cagcacaacc agcaatggcc 60
gcttcgcccg gctcc                                                  75

<210> 3
<211> 75
<212> DNA
<213> Moloney murine leukemia virus

<220>
<221> primer_bind
<222> (1)..(75)
<223> /note="Primer ecoenv18"

<400> 3

ctcgctcgcc catatgcggc cgcaggtctc ctcctcttag cagcacaacc agcaatggcc 60
caccatggac catct 75

<210> 4
<211> 27
<212> DNA
<213> Moloney murine leukemia virus

<220>
<221> primer_bind
<222> (1)..(27)
<223> /note="Primer ecoenv16"

<400> 4
agcatcacta gttttgtgtc ggttgga 27

<210> 5
<211> 27
<212> DNA
<213> Moloney murine leukemia virus

<220>
<221> primer_bind
<222> (1)..(27)
<223> /note="Primer ecoenv12"

<400> 5
agcatcacta gtcgccggtg gaagttg 27

<210> 6
<211> 27
<212> DNA
<213> Moloney murine leukemia virus

<220>
<221> primer_bind
<222> (1)..(27)
<223> /note="Primer ecoenv15"

<220>
<221> primer_bind
<222> ()..)

<400> 6
agcatcacta gtgacgcggg gtcctag 27

<210> 7
<211> 27
<212> DNA
<213> Moloney murine leukemia virus

<220>
<221> primer_bind
<222> (1)..(27)
<223> /note="Primer ecoenv13"

<400> 7

agcatcacta gtcttattat ctttgca                                          27

<210> 8
<211> 27
<212> DNA
<213> Moloney murine leukemia virus

<220>
<221> primer_bind
<222> (1)..(27)
<223> /note="Primer ecoenv14"

<400> 8
agcatcacta gttgacttgg attctcg                                          27

<210> 9
<211> 36
<212> PRT
<213> Moloney murine leukemia virus

<220>
<221> SITE
<222> (1)..(36)
<223> /Note="Amino acid sequence of ecotropic env
      variant and/or parts; NH2-terminus of constructs
      1-3 at locations 34-47"

<400> 9
Met Lys Tyr Leu Leu Pro Thr Ala Ala Ala Gly Leu Leu Leu Leu Ala
  1               5                  10                  15

Ala Gln Pro Ala Met Ala Ala Ser Pro Gly Ser Ser Pro His Gln Val
             20                  25                  30

Tyr Asn Ile Thr
        35

<210> 10
<211> 29
<212> PRT
<213> Moloney murine leukemia virus

<220>
<221> SITE
<222> (1)..(29)
<223> /Note="Amino acid sequence of ecotropic env
      variant and/or parts; COOH-terminus of construct 1
      at location 455-468"

<400> 10
Ser Tyr Val Tyr Gly Phe Glu Arg Ser Asn Arg His Lys Thr Ser Gly
  1               5                  10                  15

Gly Gly Gly Ser Pro Phe Val Cys Glu Tyr Gln Gly Gln
             20                  25

<210> 11
<211> 30
<212> PRT
<213> Moloney murine leukemia virus

<220>
<221> SITE
<222> (1)..(30)
<223> /Note="Amino acid sequence of ecotropic env
      variant and/or parts; COOH-terminus of construct 2
      at location 294-308"

<400> 11
Pro Ser Gly Thr Pro Leu Ser Pro Thr Gln Leu Pro Pro Ala Thr Ser
1               5                   10                  15

Gly Gly Gly Gly Ser Pro Phe Val Cys Glu Tyr Gln Gly Gln
            20                  25                  30

<210> 12
<211> 29
<212> PRT
<213> Moloney murine leukemia virus

<220>
<221> SITE
<222> (1)..(29)
<223> /Note="Amino acid sequence of ecotropic env
      variant and/or parts; COOH-terminus of construct 3
      at location 253-266"

<400> 12
Gly Ile Arg Leu Arg Tyr Gln Asn Leu Gly Pro Arg Val Thr Ser Gly
1               5                   10                  15

Gly Gly Gly Ser Pro Phe Val Cys Glu Tyr Gln Gly Gln
            20                  25

<210> 13
<211> 35
<212> PRT
<213> Moloney murine leukemia virus

<220>
<221> SITE
<222> (1)..(35)
<223> /Note="Amino acid sequence of ecotropic env
      variant and/or parts; NH2-terminus of constructs 4
      and 5 at location 83-95"

<400> 13
Met Lys Tyr Leu Leu Pro Thr Ala Ala Ala Gly Leu Leu Leu Leu Ala
1               5                   10                  15

Ala Gln Pro Ala Met Ala His His Gly Pro Ser Tyr Trp Gly Leu Glu
            20                  25                  30

Tyr Gln Ser
        35

<210> 14
<211> 29
<212> PRT
<213> Moloney murine leukemia virus

<220>
<221> SITE
<222> (1)..(29)
<223> /Note="Amino acid sequence of ecotropic env
      variant and/or parts; COOH-terminus of construct 4
      at location 200-213"

<400> 14
Thr Ser Asp Gln Ala Val Gln Val Cys Lys Asp Asn Lys Thr Ser Gly
  1               5                  10                  15

Gly Gly Gly Ser Pro Phe Val Cys Glu Tyr Gln Gly Gln
             20                  25

<210> 15
<211> 29
<212> PRT
<213> Moloney murine leukemia virus

<220>
<221> SITE
<222> (1)..(29)
<223> /Note="Amino acid sequence of ecotropic env
      variant and/or parts; COOH-terminus of construct 5
      at location 150-163"

<400> 15
Val Cys Pro Gly Pro His Arg Pro Arg Glu Ser Lys Ser Thr Ser Gly
  1               5                  10                  15

Gly Gly Gly Ser Pro Phe Val Cys Glu Tyr Gln Gly Gln
             20                  25